Europäisches Patentamt

European Patent Office

Office européen des brevets

(11) Publication number: **0 320 154**
**A1**

(12) **EUROPEAN PATENT APPLICATION**

(21) Application number: 88311208.8

(22) Date of filing: 25.11.88

(51) Int. Cl.4: **C12Q 1/38 , G01N 33/579**

(30) Priority: 27.11.87 GB 8727796

(43) Date of publication of application:
14.06.89 Bulletin 89/24

(84) Designated Contracting States:
CH DE FR GB IT LI

(71) Applicant: **RADIOMETER CORPORATE**
**DEVELOPMENT LIMITED**
**The Manor Manor Royal**
**Crawley, West Sussex RH10 2PY(GB)**

(72) Inventor: **Grist, Roger William**
**Castle End Hurtis Hill**
**Crowborough Sussex(GB)**

(74) Representative: **Jones, Helen M.M. et al**
**Gill Jennings & Every 53-64 Chancery Lane**
**London WC2A 1HN(GB)**

(54) **Body fluid assay and kit.**

(57) A urine screen comprises carrying out at least two separate assays on portions of a sample. One assay detects the presence of leucocytes and the other detects the presence of bacterial cells. Each assay has as the product of reaction with the species to be detected a compound and both compounds can be detected optically at the same predetermined wavelength, usually by automated apparatus. A preferred test for leucocytes uses a synthetic substrate for leucocyte elastase. A preferred test for bacteria uses a synthetic substrate for the endotoxin sensitive limulus amoebocyte lysate. Preferably the products of the hydrolytic cleavage of both substrates include the same fluorescent compound.

EP 0 320 154 A1

## BODY FLUID ASSAY PROCESS AND KIT

The present invention relates to a method for assaying a body fluid, suitably urine, to detect the presence of white blood cells (leucocytes) and bacteria in the fluid.

In hospital microbiology labs, up to 50% of samples analyses are urine samples. At least 80 to 90% of the samples tested are negative for clinically significant indications. Thorough analysis of urine samples may involve microscopy and/or culturing and is time consuming and requires skilled operators. It is therefore desirable to eliminate negative samples in order to reduce the number of samples which have to be tested by culturing and microscopy. Any screen for that purpose must be highly specific in order that, if possible, all positive samples are identified. In order to be economically viable the screen must be reasonably specific in distinguishing between positive and negative samples, in order that a high proportion of the negative samples are identified.

It is generally accepted that a high number of white blood cells in urine, for instance above 10 cells per mm, is clinically significant in the presence of high numbers of bacteria, for instance to detect asymptomatic infections or to distinguish between pathogens in the urine and contaminants. It can also be significant in the presence of lower counts of bacteria, since it may indicate inflammation without infection or infection by certain agents such as Chlamydia trachomatis, Mycoplasma spp or Neisseria gonorrheae.

Until recently the level of bacteria in a urine sample which has been taken as clinically significant evidence of infection, was above $10^5$ colony-forming units per ml sample (cfu/ml). More recently amounts of bacteria below that level have been found to be clinically significant. Although levels as low as 100 or 1000 cfu per ml of some species can produce pathological symptoms, a level which has been accepted as including the vast majority of significant samples is $10^4$ cfu/ml.

It is also accepted that diagnosis of urinary tract infection is improved, particularly for low bacterial loads, if a samples is simultaneously analysed for the presence of pyuria. A number of investigations have been conducted to correlate results of assays for bacteriuria and measurements of leucocytes levels in urine samples. In Am. J. Clin. Path. (1975) 63 p. 142, Jorgensen and Jones use the limulus assay to detect gram-negative bacteria, observing a positive result by a gelation method, together with observations of leucocytes using a gram stain under a microscope. The presence of bacteria was also observed under the microscope.

Chemstrip LN is a commercially available test strip comprising, interalia, a pad including reagents which test for the presence of leukocyte esterase in the sample and a pad containing reagents to analyse for nitrite thereby to test the presence of nitrite-forming bacteria. The step is further described in Kusumi et al in JAMA 245 1653-1655. A positive reaction is indicated by changes of colour of the pad, in both cases. The colour changes are observed visually and compared with a chart. The colour of the pads is different and this aids the observer to differentiate between the pads and compare the colours with the appropriate part of the chart.

It is difficult to automate such a system since an accurate and sensitive analysis of the pad colours would require the measurement of reflection at different wavelengths. The non-automated visual interpretation of the results requires that the observer has some degree of skill.

In EP-A-0237394 chromogenic substrates of cholinesterase are used in an assay for determining the presence of leucocytes in urine.

In EP-A-0012957 (and US-A-4278763) chromogenic indoxyl ester derivatives of amino acids and peptides are disclosed as substrates for esterases in neutrophilic leucocyte grannulocytes. They are apparently hydrolysed by proteolytic enzymes including elastase chymotrypsin and trypsin for detection of those enzymes in aqueous solutions and in body fluids other than urine.

In EP-A-0158225 (and US 4758508) substrates which are esters of phenols are used in an assay for esterolytic or proteolytic enzymes in leucocytes for instance in urine. One substrate is an alanyl ester of a phenol derivative.

Another commercially available urine analysis system is the Autotrak system in which a urine sample is contacted with a fluorescent stain, which stains bacteria as well as leucocytes. The apparatus counts the stained white blood cells and bacteria and can differentiate between the two types of cell. One problem with the Autotrak system is that the equipment is relatively expensive and is difficult to adapt for uses other than as a urine screen.

The amoebocyte lysate of horseshoe crabs is widely used to test for the presence and amount of bacterial endotoxin in samples. The gelation reaction which is triggered by endotoxins can be observed by assessing turbidity changes, (US4038029), by analysing the creation of a plug by the clot in a capillary tube (US 4370413) or by testing the depth of penetration of objects dropped onto the surface of an assay mixture. More recently synthetic substrates have been developed which are hydrolysed by products formed during the clotting process to produce for instance coloured compounds or fluorescent compounds. For instance in EP 80649 biological fluids, including urine, are analysed using synthetic peptide substrates for the amocboayte lysate, especially ones which are hydrolysed to produce p-nitroaniline, whose presence can be detected directly, by observing its colour, or which can be converted to another compound, e.g. by coupling with a coupling reagent to form an azo dye which has a more easily observable colour. Fluorogenic substrates are also mentioned. In EP224830 chromogenic or fluorogenic substrates with amoebocyte lysate of horseshoe crab are used in a urine screen.

US 4188264 discloses a range of fluorogenic and chromogenic substrates for the horshoe crab amoebocyte lysate assay.

According to the invention a new method for testing body fluid comprising carrying out two separate assays on a sample of fluid and optically assessing the results of the assays using a detector sensitive to a predetermined wavelength and comprising

a) forming a first assay mixture containing a portion of the sample of fluid and a leucocyte reagent that is reactive with leucocytes in the assay mixture to form a first compound, the presence of which can be determined in the assay mixture by said optical detector,

b) forming a second assay mixture containing a portion of the sample of fluid and a bacteria reagent that is reactive with bacteria in the assay mixture to form a second compound, the presence of which can be determined in the assay mixture by said optical detector, and

c) determining the presence of said first and second compounds by optical assessment using said detector at said predetermined wavelength.

The wavelength may be a band of wavelengths.

In the invention the detector usually comprises a light source and a light sensor. The light source illuminates the assay mixture and the light sensor detects light emitted from the mixture. The emitted light may be reflected light, transmitted light or fluorescent light produced by a fluorescent compound in the assay mixture excited by the incident light from the light source.

The predetermined wavelength to which the detector is sensitive may be a wavelength of incident light from the source and may be produced by the use of a source producing the particular wavelength or may be the result of filtering light from a source producing a broad range of wavelengths. Alternatively or additionally it may be a wavelength to which the sensor is sensitive. The emitted light from the assay mixture may be filtered to produce light of that wavelength for sensing by the sensor and/or the emitted light may be of the specific wavelength, e.g. if it is produced by the fluorescence of a component of the assay mixture. Preferably detection of the said compounds of both assay mixtures is carried out using the same excitation wavelength and the same emission wavelength. Often the excitation wavelength is different to the emission wavelength.

It is particularly preferred for the components to be detected are fluorescent and that they are detected by their fluorescence.

The method is of most use for testing urine although it is suitable also for testing other body fluids including cerebro spinal fluid, blood, sputum etc. The method of the invention is extremely useful as a screen for processing a large number of urine samples, usually largely automatically.

By using this method the screening method can be automated easily, and can use a relatively simple detector without the necessity of changing light sources, sensors or filters between readings. Automation enables unskilled operators to use the method with little training which improves efficiency for the laboratory. The method is of particularly value when the optical detection is by fluorescence detection.

Although each of the assays may be observed by continuously monitoring the assay mixtures to detect the formation of the compounds over a period of time. It is convenient to measure for the presence of the compounds in each of the assay mixtures at approximately the same time and using a single measurement only. The assays can therefore be such that the end point of the reactions is reached after about the same period of time. Preferably the optical detection is carried out after a period in the range 5 minutes to 2 hours after the commencement of the reactions.

In accordance with accepted practice for urine testing, it is preferred for the first assay to be capable of detecting the presence of more than 10 leukocyte cells per microlitre. Also in accordance with accepted practice, it is preferred for the second assay to be capable of detecting at least $10^4$ cfu/ml.

The optical detection may include the measurement of the change in absorption or of reflection by the assay mixture of light of the given wavelength or it may measure a change in the fluorescent properties of the assay mixture. The compounds which are formed by reaction of the substrate in the assay mixtures may be capable of direct detection by the optical means, for instance the compounds may be coloured differently to the reactive reagents or may have different fluorescence properties. Alternatively the compounds may be indirectly detected for instance they interact with other components of the assay mixture to form other compounds which can be detected directly. For instance they may be coupled with a coupling reagent to produce a dye, e.g. an azo dye, as described in EP 80649. Alternatively the compound or a further reaction product may affect the optical properties of another component of the assay mixture, e.g. by quenching, for instance absorbing, fluorescence emitted by another component, for instance as described in US4495293.

Preferably the compounds produced by reaction of the reagents are both capable of direct detection, since this reduces the number of components of the assay mixture. It is most convenient if the compounds produced in both assays is the same. The substrates may react by being cleared in the assay mixture, usually being hydrolysed, but can also react by being oxidised or reduced and/or take part in an addition reaction. Examples of reagents which produce coloured compounds are ester and amide derivatives of p-nitroaniline(NA), of p-diethylaminoaniline (DEAA) and of 5-nitro-$\alpha$-naphthylamine. Examples of reagents which produce fluorescent compounds are esters or amides of $\beta$-naphthylamine, of $\beta$-naphthol, of $\alpha$-naphthol, of indoxyl, of N-methyl-indoxyl, of 7-amino 4-methyl- or 7-amino-4-trifluoromethyl-coumarin, of 4-methyl- or 4-trifluoromethyl- umbelliferone, or of resorufin. Fluorogenic substrates are preferred and of the fluorogenic substrates available the most preferred are coumarin derivatives.

The first assay may comprise a reaction which detects the presence of leukocyte esterase in the sample. The reagent will thus be a substrate for leukocyte esterase which is hydrolysable to produce a detectable compound. Suitable substrates are peptidyl derivatives, e.g. esters, for instance the indoxyl ester as used in Chemstrip LN or an analog or as described in EP-A-0012952 or EP-A-0158225 or cholinesterase substrates as described in EP-A-0237394. Analogs of any of these substrates wherein the product of hydrolysis is a fluorescent compound can be used.

Preferably the leukocyte assay detects the presence of leucocyte elastase and thus uses a synthetic substrate for elastase. In the most preferred aspect of the invention the substrate's hydrolysis produces a fluorescent compound.

The elastase substrate used in such a preferred assay is a peptide derivative which is sensitive and specific for the elastase enzyme. Thus the substrate is preferably substantially incapable of being hydrolysed by other enzymes normally present in the body fluid or even by leucocyte esterase, trypsin or chymotrypsin, i.e. is hydrolysed very slowly or not at all by those other proteases. Suitable substates are compounds of the formula I

$$R^1\text{-}A^4\text{-}A^3\text{-}A^2\text{-}A^1\text{-}CONH\text{-}R^2$$

in which $A^1$ is an amino acid residue having a non-polar side chain and preferably being selected from alanyl valyl, phenylalanyl and methionyl, $A^2$ is a bond or an amino acid residue having a non-polar side chain and preferably selected from prolyl, alanyl and leucyl, $A^3$ is a bond or is an amino acid residue having a non-polar side chain and is preferably an alanyl residue and $A^4$ is a bond or is an amino acid residue having a non-polar side chain and is preferably an alanyl residue, $R^1$ is glutaryl or is an N-terminal blocking group and $R^2$ is selected such that the compound $R^2NH_2$ can be detected in the assay medium by the defined detector. Most preferred are compounds in which $A^1$ is alanyl or valyl, preferably valyl, and $A^2$ is alanyl or prolyl and $A^3$ is alanyl. The blocking group is selected from acetyl(Ac), succinyl(Suc), methoxysuccinyl (MeOSuc), t-butyloxycarbonyl (Boc) and glutaryl(Glt). MeOSuc is found to be a particularly preferred group.

There are many known peptidyl derivatives which are suitable as substrates, and these include derivatives of:

4

Ac - Ala - Ala - Ala

Ac - Ala - Ala - Pro - Ala

Ac - Ala - Ala - Pro - Phe

Ac - Ala - Pro - Ala

Boc - Ala

Boc - Ala - Ala

Boc - Ala - Ala - Ala

Boc - Ala - Ala - Pro - Ala

Boc - Ala - Ala - Pro - Val

Glt - Ala - Ala - Ala

MeOSuc - Ala - Ala - Pro - Val

Suc - Ala - Ala - Ala

Suc - Ala - Ala - Pro - Met

Suc - Ala - Ala - Val

Suc - Ala - Try - Leu - Val

Pyr - Pro - Val

The most preferable substrates are derivatives of MeOSuc - Ala - Ala - Pro - Val, which is described in Castillo et al, in Analytical Biochemistry (1979) 99, 53-64, and of Suc-Ala-Ala-Val which is described in "Methods of Enzymatic Analysis" 3rd Edn. vol.5 p176-184 by Tschesche et al and in Hoppe-Seyler's Z Physiol. Chem (1980) 361 1413-1416 by Wenzel et al.

The second assay, for bacteriuria, may be based on any of the currently accepted assays for bacteriuria. For instance it may detect nitrate-reducing bacteria utilising the Griess test using a suitable substrate. The test may be for instance as described in EP-A-171558 or FR-A-2196386. Other chemical assays for detecting bacteria are the test for catalase described in J. Lab. & Clin.Med.(1961) 490-494, and an assay using one or a cocktail of substrates hydrolysable by bacterial enzymes (e.g. phosphatases, galactosidase and peptidases), for instance as described in EP-A-91837 or EP-A-122148. Another assay utilises redox dyes which are capable of reacting with e.g. being reduced by a component of the respiratory chain of bacteria.

Preferably the second assay comprises a component mixture including an amoebocyte lysate of horseshoe crab, or an extract containing a pro clotting enzyme thereof, with a synthetic substrate which is capable of being hydrolysed in the presence of the lysate and endotoxin to produce a fluorescent compound. The assay may be carried out as described in for instance EP-A-80649, EP-A-224830 or US-A-4188264, and may use any of the fluorogenic substrates described in any of those specifications. Usually the substrate is a derivative of -Gly-Arg- in which the arginine is connected via its carboxyl group to a chromogenic or fluorogenic group, usually via a -CONH-bond and the Gly is connected to an amino acid containing group, eg an L-amino acid or peptide optionally having a blocking group or a D-amino-acid at its N-terminal.

The process can also include carrying out a third assay or additionally a fourth or even more assays, on further portions of the fluid sample, in which the reaction products can be detected by the same optical detector. For instance it may be advantageous for use as a third assay a test for the detection of nitrate-reducing bacteria, which is particularly useful for detecting gram-positive bacteria, for instance to be used in combination with the LAL test which detects gran-negative bacteria. Such a test is described in FR-A-2196386 and, preferably, a fluorescence detecting test as described in EP-A-171558 is used. A fourth assay could be one using a cocktail of substrates for detecting common hydrolytic enzymes, for instance as described in EP-A-91837 or EP-A-122148. We have found however that the combination of two test, especially the preferred leucocyte elastase and LAL tests, can be adequate for the urine screen and it is preferred to keep the method as simple as possible by having only two assays.

The sample on which each of the assays is carried out may be a direct fluid sample or may be diluted, or may be derived from fluid by centrifugation or other processing methods. It is preferred for the sample to be a direct fluid, eg urine, sample or a diluted fluid, eg urine, sample. Preferably the liquid component of each of the assay mixtures is derived substantially only from the body fluid.

It is found that in some circumstances, when carrying out the amoebocyte lysate test in a urine screen, it is preferable for the urine to be diluted, since this appears to overcome an inhibition of the clotting cascade, which can happen in about 5% of urines. The dilution may be between 5 and 500 times, and is usually between 10 and 200 times, for instance between 20 and 100 times.

There is also provided in the invention a kit for carrying out the method of the invention, comprising an assay container for each of the assay mixtures and reagents for the assays. The containers may be physically separate or, preferably, may be physically attached to each other. Preferably the containers are suitable for containing the assay mixture during the optical detection step. The containers may be in the form of tubes or of absorbent pads in the interstices of which the liquid assay mixture can be held. Such absorbent pads are preferably supported on a solid non-absorbent substrate and are preferably suitable for dipping into the sample to be tested. Another most preferred example of the kit is in the form of a plate having a plurality of wells which form the assay containers, for instance a microtitre plate having an array of columns and rows of wells. The kit may include reagents for screening a plurality of urine samples and/or for carrying out other assay(s) on the body fluid sample.

In the kit the assay containers may be provided with some or all of the necessary reagents in pre-prepared form for carrying out the respective assay process. Alternatively some of the reagents may be provided in the kit in a form in which they can be transferred into the container immediately before or during the reaction. It is most convenient for the reagents to be provided in the assay container in a form in which they can be directly reconstituted in the portion of the undiluted urine sample used in the assay mixture.

The reagents may be provided in the kit in freeze-dried, air-dried or, preferably, vacuum-dried form. The preprepared containers may also contain stabilisers for the reagents, which stabilise the components physically and chemically during production and/or storage and render them storage stable. A suitable method of stabilising reagents in containers is described in GB-A-1574707. Stabilisers are water-soluble and can be natural materials, eg proteins, for instance gelatin, or may be synthetic polymers, for instance polyvinylalcohol. They may also contain buffers.

When using the amoebocyte lysate test we have found that it can sometimes be difficult to provide the lysate and the synthetic substrate in the same container in a stable form, it being difficult to prevent the gelation cascade activating during production of the kit. It is possible to inhibit the premature activation of the cascade by the introduction of an inhibitor which acts as a stabiliser during production of the kit and during storage. This inhibitor is incorporated into the solution of either the reagent or the lysate which is subsequently dried. Alternatively or additionaly the lysate solution may be buffered to a pH at which it is substantially inactive, for instance above pH8 or 9 or below pH6.


EXAMPLE 1

The following example illustrates the invention:

Microtitre plates are prepared for assaying for endotoxin using the LAL test and leukocyte elastase. Into a portion of the wells (LAL wells) a solution comprising lyophilised lysate of limulus amoebocyte (obtained from Cape Cod Associates, U.S.A.), pyrogen free water and a buffer is deposited into the well and the solution is then dried. The amount of solution that is deposited is such that when reconstituted for use, the lysate will be diluted around 30 times compared to the normal concentration at which it is used.

Other (elastase) wells are supplied with a reagent solution containing 380ml of a 5% phosphate buffer at pH of 7.5, 200ml of a 15%w/v PVA made up in distilled and deionised water, l2gm of Saponin (Sigma) and a solution of 1gm of elastase substrate (MeOSuc-Ala-Ala-Pro-Val-AMC) in 100ml of 2Me (BDH Analar). 50 microlitres of the solution is dosed into the well and dried under reduced pressure.

These prepared plates were then used in a urine assay. For the LAL wells the reagent mixture in the wells is reconstituted with 50 microlitres of a solution containing a buffer and the LAL substrate BOC-Val-Gly-Arg-7AMC in the desired amount. The urine sample to be tested is added to that well by transferring 1 microlitre of the urine using a sterile pyrogen-free loop.

The elastase well is reconstituted directly with the undiluted urine sample.

The plate is incubated at 37° C for 30 minutes and then the fluorescence emission by the hydrolysed 7 AMC in the wells is measured in a fluorimeter. The urine samples were also analysed by microscopic techniques to find the leucocyte count and by normal culture techniques to assess the microorganism level. The results of the microscope and culture tests are shown in table 1 and the fluorimeter readings and their interpretation as positive or negative urine samples in shown in table 2.

TABLE 1

| | | Culture result (cfu/ml) | |
|---|---|---|---|
| urine sample | Leucocyte count | Gram + ve | Gram - ve |
| 1 | 20 | $10^4$ | - |
| 32 | 300 | $>10^4$ | - |
| 21 | 0 | - | $>10^5$ |
| 18 | 3 | - | $>10^5$ |
| 25 | 200 | - | $>10^5$ |
| 27 | 300 | - | $>10^5$ |
| 4 | 250 | - | - |
| 16 | 100 | - | - |
| 2 | - | - | - |
| 6 | - | - | - |

TABLE 2

| Urine sample | Leucocyte test (elastase) | LAL test | Screen interpretation | | |
|---|---|---|---|---|---|
| | | | Leuco | LAL | Overall |
| 1 | 1050 | 2125 | + | + | + |
| 32 | 4092 | 1210 | + | - | + |
| 21 | 537 | 3159 | - | + | + |
| 18 | 572 | 3536 | - | + | + |
| 25 | 9999 | 9999 | + | + | + |
| 27 | 9999 | 2822 | + | + | + |
| 4 | 9999 | 1971 | + | + | + |
| 16 | 2644 | 1551 | + | - | + |
| 2 | 454 | 1285 | - | - | - |
| 6 | 665 | 1727 | - | - | - |

Results over 1842 counts are interpreted as positive. A count of 9999 is off scale.

In isolation the leucocyte test would have failed to detect samples 21 and 18. The LAL test would have failed on 32 and 16. However, with a combination of the two all positive samples are detected, but the two negative samples are correctly screened out.

EXAMPLE 2

Another plate is prepared including LAL wells and elastase wells. The LAL wells are prepared as follows:

Freeze-dried LAL in an amount which is suitable for preparing 5ml of a 0.125 Eu/ml sensitivity solution (produced by Associates of Cape Cod) is dissolved in 160ml of 0.05% solution of polyvinylalcohol (cold water soluble-produced by Sigma). 50µl of the solution is dosed into the wells and the solutions vacuum dried.

The elastase wells are prepared by the following procedure.

A buffer solution was made up using 36.15g disodium hydrogen orthophosphate (anhydrous), 4.0g potassium dihydrogen orthophosphate and 9.0g CHAPS in 800 ml water (distilled and deionised). The solution was made up to 980 ml. A solution of 2g the same elastase substrate used in the previous

7

examples was made up in 20 ml DMSO and added to the buffer. 5.0g Rhizopus peptidase was dissolved in the substrate solution. 50 $\mu$l portions of the solution were dosed into the wells of a microtitre plate and the solutions dried by vacuum drying.

The plates also have a set of empty wells and a set of "blank wells". The plates are then used as follows:

Firstly a LAL substrate stock solution is made up by mixing 250 ml pyrogen free water, 1.25g sodium hydroxide, 9.38g TES biological buffer (Sigma) 2.5g polyvinyl alcohol (as above) and 1.25g magnesium chloride. The mixture is heated to dissolve the ingredients, adjusted to pH 8.00 ± 0.05 using pyrogen free 0.1 MHCl or 0.1 MNaOH. The solution is then filter sterilised.

Separately a solution of Boc-Val-Gly-Arg-AMC is made up to 0.1% w/v in 2-methoxyethanol. 300$\mu$l of that solution is mixed with 9.7ml of the buffer. Immediately before use 50$\mu$l of the substrate stock solution is added to the wells containing dried LAL.

50$\mu$l portions of a urine sample are added to one empty well, one blank well and one elastase well. 1$\mu$l of urine is transferred using a sterile plastic loop from the urine added to the empty well to the LAL well. The timing of the assay starts at that point.

Portions of other urine samples are dosed into other wells on the same plate. The plate is covered with an adhesive sheet seal and incubated at 37°C for 30 minutes, after which the presence of 7-amino-4-methyl-coumarin is detected in each of the wells fluorimetrically by an automatic reader. The readings are compared with the neat urine reading in the blank wells. The tests register positive when the urine contains more than $10^4$ cfu/ml bacteria or $10^4$ leucocytes /ml respectively. If there is a positive result from either or both tests the urine is subjected to further tests, for instance to identify any infection and/or determine the antibiotic susceptibility of the infection. If both tests are negative then the urine does not need to be subjected to further tests.

## EXAMPLE 3

In another embodiment of the LAL test, wells were prepared as follows:

A pH5.8 buffer was prepared by mixing 2 parts (by volume) of a 0.1M solution of a citric acid in pyrogen free water with 3 parts of a 0.2M solution of disodium hydrogen orthophosphate. Freeze dried LAL in an amount which is suitable for preparing 5 ml of a 0.125 Eu/ml sensitivity solution (produced by Whittaker) was re-dissolved in 10 ml of a 0.1% polyvinylalcohol solution. To this was added 10 ml of the pH5.8 buffer. 50$\mu$l portions of the solution were dosed into wells of a microtitre plate and vacuum dried.

The plates were subsequently used as in Example 2, i.e. including wells with elastase substrate and reconsituting the LAL with the same substrate stock solution and adding the urine portions in the same manner. The pH of the reaction mixture in the urine well is about 7.3.

## Example 4

In another embodiment of the LAL wells, the following solutions are made up. 50$\mu$l portion of a substrate solution as in Example 2, but containing also 0.05% poly-vinyl alcohol and buffered at pH8.00 ± 0.05 were dosed into wells of a plate and dried. A buffered pH5.8 lysate solution, was made up immediately before use of the kit, by the same method as example 3, but omitting the polyvinyl alcohol.

The wells are reconstituted by adding 50$\mu$l portions of the lysate solution to the substrate containing wells to give a pH of about 7.3, and then 1$\mu$l urine is added in the same way as in the preceding examples.

## Claims

1. A method for testing a body fluid which comprises carrying out two separate assays on a sample of the fluid and optically assessing the results of the assays using a detector sensitive to a predetermined wavelength and comprising:

a) forming a first assay mixture containing a portion of the sample of fluid and a leucocyte reagent that is reactive with leucocyte in the assay mixture to form a first compound, the presence of which can be determined in the assay mixture by said optical detector,

b) forming a second assay mixture containing a portion of the sample of fluid and a bacteria reagent that is reactive with bacteria in the assay mixture to form a second compound, the presence of which can be determined in the assay mixture by said optical detector, and

c) determining the presence of said first and second compounds by optical assessment using said detector at said predetermined wavelength.

2. A method according to claim 1 in which the fluid is urine.

3. A method according to claim 1 or 2 in which the predetermined wavelength is a wavelength of incident light from the light source of the detector.

4. A method according to any preceding claim in which the predetermined wavelength is a wavelength to which the sensor of the detector is sensitive.

5. A method according to any preceding claim in which light emitted from each assay mixture that is detected by the sensor of the detector is fluorescent light.

6. A method according to any preceding claim in which said first compound and said second compound are the same, preferably being fluorescent, more preferably being a fluorescent coumarin derivative.

7. A method according to any preceding claim in which the leukocyte reagent is a substrate for leukocyte elastase, and is preferably a derivative of MeOSuc-Ala-Ala-Pro-Val.

8. A method according to any preceding claim in which the bacteria reagent comprises an amoebocyte lysate of horseshoe crab, or an extract containing a pro-clotting enzyme thereof, and a synthetic lysate substrate hydrolysable in the presence of the lysate and endotoxin, the substrate preferably being a derivative of BOC - Val - Gly - Arg.

9. A kit suitable for carrying out a method according to any preceding claim, comprising an assay container for each of the assay mixtures, a leukocyte reagent and a bacteria reagent, respectively as defined in claim 1.

10. A kit according to claim 9 in which the assay containers for the first and second assay mixtures are physically attached to each other, preferably being wells of plate having a plurality of wells.

11. A kit according to either claim 9 or 10 in which the assay containers are provided with at least one of the reagents in preprepared form, preferably in the form of a dried film on the internal surface of the respective container.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | ANALYTICAL BIOCHEMISTRY, vol. 99, no. 1, 1979, pages 53-64, Academic Press Inc., San Diego, US; M.J. CASTILLO et al.: "Sensitive substrates for human leukocyte and porcine pancreatic elastase: A study of the merits of various chromophoric and fluorogenic leaving groups in assays for serine proteases" * Pages 53-54; page 60, column 1, lines 28-48 * | 1,5-7 | C 12 Q 1/38 G 01 N 33/579 |
| Y | Idem | 1,6,8 | |
| D,Y | DE-A-2 740 323 (SEIKAGAKU KOGYO CO.) * Claims; pages 11,12; page 14, lines 20-25 * | 1,6,8 | |
| D,Y | EP-A-0 224 830 (MILES LABORATORIES) * Abstract; claims; page 6, lines 30-58; page 7, lines 1-46 * | 1,6,8 | |
| D,Y | EP-A-0 080 649 (SEIKAGAKU) * Pages 10,11,20 * | 1,6,8 | TECHNICAL FIELDS SEARCHED (Int. Cl.4) C 12 Q G 01 N |
| D,A | EP-A-0 122 148 (AJINOMOTO CO. INC.) * Pages 1-3; page 8, claims * | 1,5,6 | |
| A | EP-A-0 056 210 (PHARMINDUSTRIE) * Claims * | 1,5,8 | |
| A | EP-A-0 074 761 (MALLINKRODT INC.) * Claims * | 1,6,7 | |

The present search report has been drawn up for all claims

| Place of search | Date of completion of the search | Examiner |
|---|---|---|
| THE HAGUE | 13-03-1989 | MEYLAERTS H. |